# EUROPEAN PATENT APPLICATION

(11) **EP 2 072 009 A1**
(43) Date of publication of application: **24.06.2009**
(21) Application number: 08172248.0
(22) Date of filing: 19.12.2008
(51) Int. Cl.: A61B 5/0408

(54) **An Electrocardiogram Sensor**

(30) Priority: 21.12.2007 NZ 56471607
(71) Applicant: Russell, Brian Keith, Auckland (NZ); Woodward, Jonathan, Auckland (NZ); Solomon, Christopher Michael, Auckland (NZ); Mallinson, Paul Benjamin, Auckland (NZ)
(72) Inventor: Russell, Brian Keith, Auckland (NZ); Woodward, Jonathan, Auckland (NZ); Solomon, Christopher Michael, Auckland (NZ); Mallinson, Paul Benjamin, Auckland (NZ)
(74) Representative: Higgs, Jonathan

(57) **Abstract**

A garment (2) comprising an integrated electrocardiogram sensor. The garment includes an electrode (8) arranged to contact a user's skin (10) and a resilient compressible filler (6) provided between the garment and the electrode such that, in use, the electrode is held substantially in place against the skin when the garment moves relative to the user's skin.

## Description

### FIELD OF THE INVENTION

The present invention relates generally to a sensor for detecting electrocardiogram signals, and particularly, although not exclusively, to electrocardiogram sensors integrated into a garment.

### BACKGROUND TO THE INVENTION

In electrocardiogram analysis, electrodes are placed against a subject's skin to detect particular electromechanical signals indicative of the subject's physiology, such as heart rate. When a subject becomes active the electrodes can move relative to the skin. This movement can cause false signals to be detected which can mask the desired ECG signal. Often ECG electrodes are integrated into clothing and as a subject moves and twists his body, the garment, and therefore the electrodes, can move relative to the skin.

It is an object of the present invention to provide an improved electrocardiogram sensor, or at least to provide the public with a useful choice.

### SUMMARY OF THE INVENTION

In a first aspect the invention broadly consists in a garment comprising:
at least one electrocardiogram sensor integrated into the garment comprising:
   an electrode on the inside of the garment and arranged to contact a user's skin; and
   a resilient compressible filler provided between the garment and the electrode such that in use the electrode is held substantially in place against the user's skin when the garment moves relative to the user's skin.

Preferably, the electrode is formed from an electrically conductive material. More preferably in a first form, the electrically conductive material is a conductive lycra. More preferably in a second form, the electrically conductive material is a conductive rubber.

Preferably, the electrode forms a pocket on the garment that is arranged to contain the resilient compressible filler.

Preferably, the electrocardiogram sensor is arranged to sense the heart rate of the user and the electrocardiogram signal of the user.

Preferably, the garment comprises an electronics module connected to the electrocardiogram sensor and arranged to receive signals indicative of the user's heart rate, or electrocardiogram signal, or both from the electrocardiogram sensor. More preferably in a first form, the electronics module is arranged to transmit or store information indicative of the user's heart rate, or electrocardiogram signal, or both. More preferably in a second form, the electronics module is provided in a hermetically sealed case.

Preferably in a first form, the resilient compressible filler has a square or rectangular cross-section.

Preferably in a second form, the resilient compressible filler has a castellated cross-section.

In a second aspect the invention broadly consists in a method of manufacturing a garment comprising an integrated electrocardiogram sensor comprising the steps of:
attaching an electrode to the inside of the garment so that the electrode is arranged to contact a user's skin;
providing a resilient compressible filler between the electrode and the garment such that in use the electrode is held substantially in place against the user's skin when the garment moves relative to the user's skin.

In a third aspect the invention broadly consists in an electrocardiogram sensor for use in a garment comprising:
an electrode arranged to contact a user's skin; and
a resilient compressible filler arranged to fit between the electrode and the garment such that in use the electrode is held substantially in place against the user's skin when the garment moves relative to the user's skin.

Preferably, the electrode is formed from an electrically conductive material.

The term "comprising" as used in this specification and claims means "consisting at least in part of". When interpreting each statement in this specification and claims that includes the term "comprising", features other than that or those prefaced by the term may also be present. Related terms such as "comprise" and "comprises" are to be interpreted in the same manner.

The invention consists in the foregoing and also envisages constructions of which the following gives examples only.

### BRIEF DESCRIPTION OF THE DRAWINGS

Preferred embodiments of the invention will be described by way of example only and with reference to the drawings, in which:
Figure 1a is a cross-sectional elevation view of a first preferred form electrocardiogram sensor of the invention;
Figure 1b is a mechanical model of the electrocardiogram sensor shown in Figure 1;
Figure 2 is a detail cross-sectional elevation view of a second preferred form electrocardiogram sensor of the invention;
Figure 3 is a perspective view of a third preferred form electrocardiogram sensor of the invention;
Figure 4 is a schematic view of a first preferred embodiment orientation and position of electrodes on a garment;
Figure 5a is a schematic view of a second preferred embodiment orientation and position of electrodes on a garment;
Figure 5b is a view of the relative positioning between the electrodes of Figure 5a and a user's ribs;
Figure 6 is a perspective cutaway view of an electronics module in communication with the electrocardiogram sensor of the invention; and
Figure 7 is a schematic view of one form of a connection between an electrode and an electronics module of the invention.

### DETAILED DESCRIPTION OF PREFERRED FORMS

The invention generally relates to an electrocardiogram (ECG) sensor used for sensing a user's heart rate, or ECG signal, or both. The ECG sensor is integrated into a garment. The ECG sensor comprises at least one electrode that is arranged to contact the user's skin. A resilient compressible filler is provided between the electrode and the garment. The resilient compressible filler provides shear movement isolation between the electrode and the skin in times when the garment moves relative to the skin.

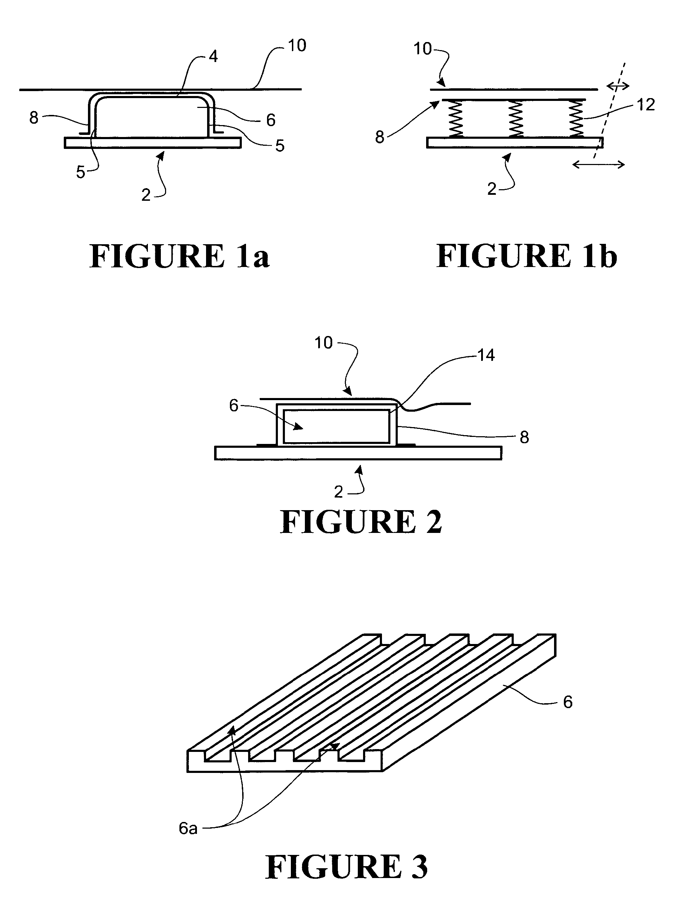

Referring to Fig 1a, a first preferred embodiment ECG sensor is integral with a garment 2 to be worn by a user. The ECG sensor may be integrated into a garment 2 during the manufacture of the garment 2. Alternatively, the ECG sensor may be integrated into a garment 2 after manufacture of the garment 2. The garment may be any suitable item of clothing or exercise accessory such as a t-shirt, shirt, singlet, tank-top, or band but is preferably worn on the user's torso. The garment 2 may be made from a fabric such as cotton, however any suitable fabric may be used. A resilient compressible filler 6 is provided on the inside of the garment 2. In this embodiment, the resilient compressible filler 6 may have a rectangular cross section with rounded or truncated corners. The resilient compressible filler 6 has one or more sides covered by an electrode 8. The electrode 8 is arranged to be in physical contact with the user's skin 10. Preferably, the resilient compressible filler 6 is made from foam, however any suitable material such as rubber, or liquid may be used. The resilient compressible filler 6 may be a woven structure, or a structure containing pockets of air. The resilient compressible filler 6 has mechanical characteristics that provides sheer movement insulation so that when the garment 2 moves relative to the user's skin, the electrode 8 does not move relative to the user's skin. Preferably, the electrode 8 is made from a conductive fabric or material, however any suitable electrical conductor such as a conductive rubber, a conductive lycra, or a conductive plastic may be used. In one form the fabric or material may be woven and in this form some or all of the strands are made from an electrically conductive material. The resilient compressible filler 6 provided between the garment 2 and the electrode 8. Preferably, the electrode 8 covers at least the top face 4 of the resilient compressible filler 6, which is the side that is closest to the user's skin 10. The electrode 8 may extend down the side faces 5 of the resilient compressible filler 6. The electrode 8 may be attached to the garment 2 such that the electrode 8 and the garment 2 form a pocket. The resilient compressible filler 6 may be held in the pocket between the electrode 8 and garment 2. In one form, the electrode 8 may be attached to the garment 2 by sewing, using glue, welding, using hook and loop fabric or using any other suitable attachment method or apparatus. Alternatively in a second form, pockets of conductive fabrics may be attached on the inner surface of the garment 2, where the conductive fabric forms the electrode 8. An opening may be provided in the garment 2 opposite the pocket of conductive fabric so that the resilient compressible filler 6 can be inserted into the pocket. The conductive fabric may act as the electrode 8. The pocket can then be closed with a zip or in any other suitable manner. This second form has the advantage that the resilient compressible filler 6 can be replaced if it loses its shape, resiliency, or any other suitable characteristic due to wear and tear. In both forms, the electrode 8 may fully or partially encase the resilient compressible filler 6.

The electrode 8 may be arranged to detect a user's heart rate, ECG signal, or both, or any other suitable physiological characteristic. Generally, at least two electrodes may be provided and placed against a user's skin in order to detect an ECG signal. A pair of electrodes, with one on each side of the heart, may measure the activity of different parts of the heart muscle. In a first form, the electrode 8 may be made entirely from a conductive material. In a second form, the electrode 8 may be made from a combination of compressible, non-conductive, and conductive materials,

Referring to Figs 1a and 1b, the resilient compressible filler may be mechanically modelled by a series of springs 12. The springs 12 may represent the resiliency and shear movement allowed by the resilient compressible filler material 6. The springs 12 may be biased so that if they are compressed they may return to their original shape. The top of the springs 12 may be able to move horizontally relative to the bottom of the springs 12, for example if a sideways force is placed on the electrode 8. In use, a user may wear the garment 2 in an active situation such as during exercise. As the user moves and rotates their body, the garment 2 can move relative to their skin 10. The resilient compressible filler 6 (represented mechanically by the springs 12) is preferably under some compression when the garment 2 is being worn. This may be achieved by using a tight-fitting garment 2 on the user. This means that the resilient compressible filler 6 will push the electrode 8 into contact with the skin 10. As the garment 2 moves relative to the skin 10, the shear movement allowed by the resilient compressible filler 6, in combination with the compression provided by the resilient compressible filler 6, may hold the electrode 8 in one place against the user's skin 10. The electrode 8 has shear isolation from the movement of the garment 2. The friction between the electrode 8 and the skin 10 also help to avoid electrode-skin relative movement.

Referring to Fig 2, a second preferred embodiment ECG sensor comprises a resilient compressible filler 6 with a rectangular cross-section. In this embodiment, when the garment 2 (and resilient compressible filler 6 and electrode 8) attempts to move relative to the skin 10, the corner 14 of the resilient compressible filler 6 may dig into the skin 10 (through the electrode 8) to create more resistance and friction between the electrode 8 and the skin 10. The compression of the resilient compressible filler 6 against the skin 10 may help to create friction, as explained above. For example, if the garment 2 tries to move right relative to the skin 10, a crease of skin 10 may form on the top right corner of the resilient compressible filler 6 which may obstruct the relative movement. The resilient compressible filler 6 may also have a square cross section.

Referring to Fig 3, a third preferred embodiment ECG sensor comprises a resilient compressible filler 6 with a castellated cross-section with many independent surfaces 6a. A single electrode 8 may cover the entire surface of the resilient compressible filler 6. Alternatively, many independent electrodes 8 may be provided on the independent surfaces 6a of the resilient compressible filler 6. As there are many independent surfaces 6a of resilient compressible filler 6 compressed into the skin 10, there is a higher chance that at least one independent surface 6a will be in good contact with the skin 10 at all times. In this third preferred embodiment, the sharp edges of the independent surfaces 6a may dig into the skin 10 in a similar manner as described for the second preferred embodiment. As there are more surfaces 6a abutting the skin 10, more friction may be provided between the electrode 8 and the skin 10 to prevent electrode-skin relative movement further. Any suitable resilient compressible filler 6 shape may be used.

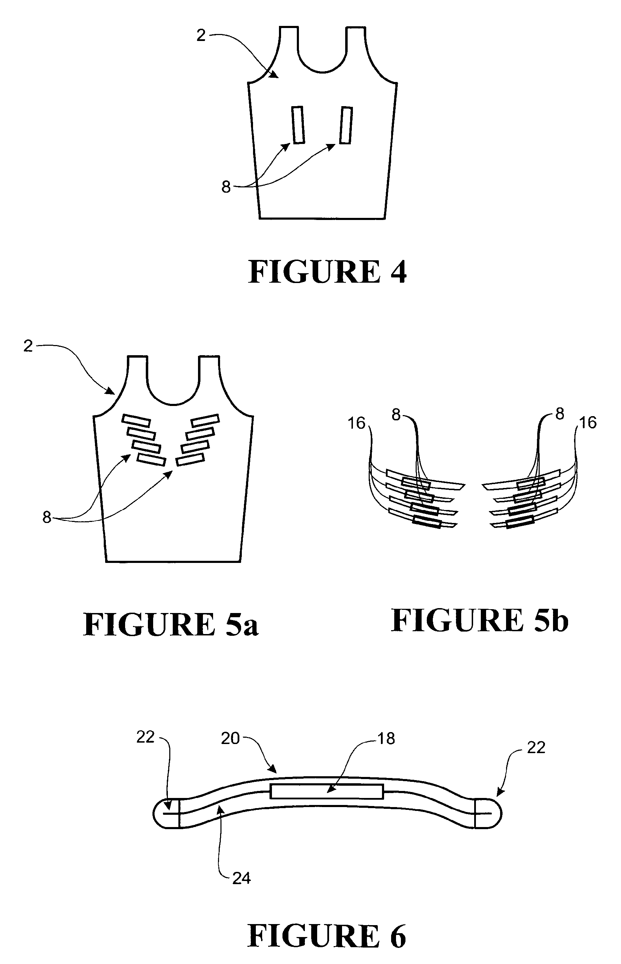

Referring to Fig 4, a first preferred embodiment orientation of the electrodes 8 is shown. The inside of a front panel of a garment 2 may comprise two electrodes 8 and corresponding resilient compressible filler (not shown) attached thereto. The electrodes 8 are provided in a substantially vertical orientation. In this embodiment, the electrodes 8 may contact the skin 10 covering the rib cage of a user. As different users may have different body shapes and sizes, this vertical orientation of the electrodes 8 may mean that many different users can use garments 2 manufactured in the same way. Even for users of different body shapes and sizes, the vertical orientation of the electrodes 8 may mean that at least some portion of the electrode 8 will be in firm contact with the user's skin 10. The electrodes 8 may be positioned over any suitable part of the user's skin 10 including the front, rear, or sides of the torso or any other suitable body part.

Referring to Fig 5a, a second preferred embodiment orientation of the electrodes 8 is shown. The inside of the front panel of a garment 2 is shown with eight electrodes 8 and corresponding resilient compressible filler (not shown) attached thereto. The electrodes 8 are provided between a substantially horizontal orientation and diagonal orientation. Referring to Fig 5b, the electrodes 8 may be positioned and orientated so as to make contact with the skin 10 above a user's individual ribs 16. The electrodes 8 may be positioned over any suitable part of the user's skin 10 including the front, rear, or sides of the torso or any other suitable body part. Referring to Figs 5a and 5b, in a first form, if multiple electrodes 8 are provided in this manner, they may be interconnected with a conductive thread, or with a single piece of conductive fabric, or in any other suitable manner. In a second form, the individual electrodes 8 may be may be electrically isolated. In this second form the electrode(s) 8 that makes the best skin contact with a user can be selected and the other electrode(s) 8 not used. Multiple electrodes 8 may also be provided by using a castellated shape (as shown in Fig 3).

Referring to Fig 6, the ECG sensor comprises an electronics module 18 arranged to receive signals indicative of the user's heart rate, ECG signal, or both from the electrodes 8. Preferably, the electronics module 18 is electrically connected to the electrodes 8. Preferably, the electronics module 18 is battery powered, however any suitable power supply may be used. Preferably, the electronics module 18 comprises a controller such as a microcontroller or a microprocessor. The controller 18 may be arranged to receive the signals and process them into information about the user's heart rate, ECG signal, or both. This may be done with a digital signal processing unit which may be provided on the electronics module 18 or as part of the controller. In a first form, the electronics module 18 may comprise a memory, such as flash memory, random access memory, a hard drive, or any other suitable memory. The signals or information indicative of the user's heart rate, ECG signal, or both may be saved into the memory for analysis at a later time. In a second form, the electronic module 18 may contain a wireless transmitter that is arranged to transmit the signals or information indicative of the user's heart rate, ECG signal, or both to a receiver for analysis. Alternatively, the electronics module 18 may be able to store and transmit the information or signals.

The electronics module 18 is preferably provided in a hermetically sealed package 20 which is shaped to fit around a user's body. Preferably, the package 20 is made from plastic or rubber. In a first form, the hermetically sealed package 20 may be connected to the electrodes 8 through a conductive thread, wire, or conductive material that runs from the electrodes 8 to the package 20. In a second form, the hermetically sealed package 20 may comprise end caps 22 that are arranged to be attached to the electrodes 8. The end caps 22 may or may not be conductive depending on the connection method as described below. A conductor 24 may provide an electrical connection between an end cap 22 and the electronics module 18. The conductor 24 may be a wire or a conductive sheet or any other suitable conductor.

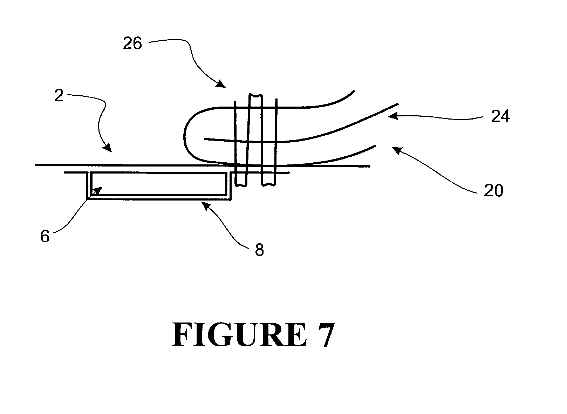

Connection of the electronics module 18 to an electrode 8 through an end cap 22 will be described. In a first form, the end cap 22 is made from a conductive material, such as metal, a conductive plastic, or any other suitable conductive material. In this first form, the electrode 8 may be welded directly onto the end cap 22. Alternatively, the electrode 8 may be adhered directly onto the end cap 22 using a conductive adhesive. Any other suitable conductive connection between the electrode 8 and end cap 22 may be used. Referring to Fig 7, in a second form, the end cap 22 is made from a non-conductive material, such as plastic, rubber, or any other suitable material. In this second form, the electrode 8 may be sewn with a conductive thread 26 to the end cap 22, the conductive thread being also sewn directly through the conductor 24. Alternatively, a conductive hook and eye fastener may be used. The hook or the eye may be conductively sewn or riveted or otherwise attached onto the electrode 8. The complimentary part of the fastener may be attached to the end cap 22 but is also conductively sewn or riveted or otherwise attached to the conductor 24. In this form the electronics module 18 may easily be removed from the garment 2 by disengaging the hook and eye. In an alternative form, the hook and eye fastener may be replaced with a conductive snap fastener where the connection method is similar. In an alternative form, the hook and eye fastener may be replaced with conductive hook and loop fabric where the connection method is similar. Any other suitable connection between the electrode 8 and the end cap 22 may be used. In Figure 7 the hermetically sealed package 20 is shown to be on the outside of the garment 2 but it may alternatively be provided on the inside.

The foregoing description of the invention includes preferred forms thereof. Modifications may be made thereto without departing from the scope of the invention as defined by the accompanying claims.

## Claims

**1.** A garment comprising:
at least one electrocardiogram sensor integrated into the garment comprising:
an electrode on the inside of the garment and arranged to contact a user's skin; and
a resilient compressible filler provided between the garment and the electrode such that in use the electrode is held substantially in place against the user's skin when the garment moves relative to the user's skin.

**2.** A garment according to claim 1 where the electrode is formed from an electrically conductive material.

**3.** A garment according to claim 2 where the electrically conductive material is a conductive lycra.

**4.** A garment according to claim 2 where the electrically conductive material is a conductive rubber.

**5.** A garment according to any one of claims 1 to 4, where the electrode forms a pocket on the garment that is arranged to contain the resilient compressible filler.

**6.** A garment according to any one of claim 1 to 5 where the electrocardiogram sensor is arranged to sense the heart rate of the user and the electrocardiogram signal of the user.

**7.** A garment according to any one of claims 1 to 6 comprising an electronics module connected to the electrocardiogram sensor and arranged to receive signals indicative of the user's heart rate, or electrocardiogram signal, or both from the electrocardiogram sensor.

**8.** A garment according to claim 7 where the electronics module is arranged to transmit or store information indicative of the user's heart rate, or electrocardiogram signal, or both.

**9.** A garment according to claim either of claims 7 or 8 where the electronics module is provided in a hermetically sealed case.

**10.** A garment according to any one of claims 1 to 9 where the resilient compressible filler has a square or rectangular cross-section.

**11.** A garment according to any one of claims 1 to 9 where the resilient compressible filler has a castellated cross-section.

**13.** A method of manufacturing a garment comprising an integrated electrocardiogram sensor comprising the steps of:
attaching an electrode to the inside of the garment so that the electrode is arranged to contact a user's skin;
providing a resilient compressible filler between the electrode and the garment such that in use the electrode is held substantially in place against the user's skin when the garment moves relative to the user's skin.

**14.** An electrocardiogram sensor for use in a garment comprising:
an electrode arranged to contact a user's skin; and
a resilient compressible filler arranged to fit between the electrode and the garment such that in use the electrode is held substantially in place against the user's skin when the garment moves relative to the user's skin.

**15.** An electrocardiogram sensor according to claim 14 where the electrode is formed from an electrically conductive material.
